(19) **Europäisches Patentamt**
**European Patent Office**
**Office européen des brevets**

(11) **EP 4 772 027 A1**

(12) **EUROPEAN PATENT APPLICATION**
published in accordance with Art. 153(4) EPC

(43) Date of publication:
**08.07.2026 Bulletin 2026/28**

(51) International Patent Classification (IPC):
*A23L 33/135* (2016.01)      *A61K 35/744* (2015.01)
*A61P 3/10* (2006.01)        *A61P 25/00* (2006.01)
*A61P 35/00* (2006.01)       *A61P 43/00* (2006.01)
*C12N 1/20* (2026.01)

(21) Application number: **24859987.0**

(22) Date of filing: **30.08.2024**

(52) Cooperative Patent Classification (CPC):
**A23L 33/135; A61K 35/744; A61P 3/10;**
**A61P 25/00; A61P 35/00; A61P 43/00; C12N 1/20**

(86) International application number:
**PCT/JP2024/031324**

(87) International publication number:
**WO 2025/047962 (06.03.2025 Gazette 2025/10)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **31.08.2023 JP 2023141141**

(71) Applicant: **TOHOKU UNIVERSITY**
**Sendai-shi, Miyagi 980-8577 (JP)**

(72) Inventors:
• **KITAZAWA, Haruki**
  **Sendai-shi, Miyagi 980-8577 (JP)**
• **NISHIYAMA, Keita**
  **Sendai-shi, Miyagi 980-8577 (JP)**
• **NAMAI, Fu**
  **Sendai-shi, Miyagi 980-8577 (JP)**

(74) Representative: **Winter, Brandl - Partnerschaft**
**mbB**
**Alois-Steinecker-Straße 22**
**85354 Freising (DE)**

(54) **MITOCHONDRIAL-FUNCTION-IMPROVING AGENT**

(57)    The problem to be solved by the present invention is to provide a novel mitochondrial-function-improving agent. The present invention provides a mitochondrial-function-improving agent comprising lactic acid bacteria capable of suppressing the production of reactive oxygen species in mitochondria.

**Description**

Technical Field

The present invention relates to a mitochondrial-function-improving agent.

Background Art

Mitochondria are essential organelles for energy production through cellular respiration. In cellular respiration, oxygen serves as an electron acceptor, generating reactive oxygen species (ROS). The ROS production level in mitochondria is usually regulated by the function of the mitochondria themselves; however, it is known that the ROS production level increases due to mitochondrial dysfunction caused by age-related cellular senescence and inflammatory stress (NPL 1). It has also been reported that the increased ROS production level in mitochondria is associated with the onset and progression of diseases, such as neurodegenerative diseases, diabetes, and cancer (NPL 2 to NPL 4). In particular, in Japan, where increasing social security costs for the elderly are regarded as a problem, there is a demand for the development of methods for preventing and treating these diseases.

Citation List

Patent Literature

PTL 1: WO2021/241728

Non-patent Literature

NPL 1: Biomedicines 9, 216 (2021)
NPL 2: Cell 166, 555-566 (2016)
NPL 3: The World Journal of Men's Health 40, 399 (2022)
NPL 4: International Journal of Molecular Sciences 22, 6479 (2021)

Summary of Invention

Technical Problem

A problem to be solved by the present invention is to provide a novel mitochondrial-function-improving agent.

Solution to Problem

Under such circumstances, the present inventors found that lactic acid bacteria capable of suppressing the production of ROS improve mitochondrial function. The present invention is based on such novel findings.
Accordingly, the present invention provides the following items.
Item 1. A mitochondrial-function-improving agent, method, use, lactic acid bacteria for use, or a composition as stated in any one of the following Items 1-1 to 1-5.
Item 1-1. A mitochondrial-function-improving agent comprising lactic acid bacteria capable of suppressing the production of reactive oxygen species in mitochondria.
Item 1-2. A method for improving mitochondrial function in a subject in need thereof, comprising administering to the subject an effective amount of lactic acid bacteria capable of suppressing the production of reactive oxygen species in mitochondria.
Item 1-3. Use of lactic acid bacteria capable of suppressing the production of reactive oxygen species in mitochondria, for the manufacture of a mitochondrial-function-improving agent.
Item 1-4. Lactic acid bacteria capable of suppressing the production of reactive oxygen species in mitochondria, for use in improving mitochondrial function.
Item 1-5. A composition for improving mitochondrial function, comprising lactic acid bacteria capable of suppressing the production of reactive oxygen species in mitochondria.
Item 2. The mitochondrial-function-improving agent, the method, the use, the lactic acid bacteria for use, or the composition according to Item 1, wherein the lactic acid bacteria reduce the production level of reactive oxygen species in mitochondria to 70% to 90%.

Item 3. The mitochondrial-function-improving agent, the method, the use, the lactic acid bacteria for use, or the composition according to Item 1 or 2, wherein the lactic acid bacteria comprise *Ligilactobacillus salivarius.*

Item 4. The mitochondrial-function-improving agent, the method, the use, the lactic acid bacteria for use, or the composition according to any one of Items 1 to 3, wherein the lactic acid bacteria comprise *Ligilactobacillus salivarius* with accession number NITE BP-03218 or *Ligilactobacillus salivarius* with accession number NITE ABP-03958, or both.

Item 5. The mitochondrial-function-improving agent, the method, the use, the lactic acid bacteria for use, or the composition according to any one of Items 1 to 4, wherein the lactic acid bacteria are capable of suppressing the production of reactive oxygen species in mitochondria in intestinal epithelial cells or intestinal macrophages.

Item 6. The mitochondrial-function-improving agent, the method, the use, the lactic acid bacteria for use, or the composition according to any one of Items 1 to 5, wherein the improvement of mitochondrial function includes at least one selected from the group consisting of an increase in ATP production level and an increase in mitochondrial oxygen consumption rate.

Item 7. The mitochondrial-function-improving agent, the method, the use, the lactic acid bacteria for use, or the composition according to any one of Items 1 to 6, wherein the mitochondrial-function-improving agent is a food or a beverage.

Item 8. The mitochondrial-function-improving agent, the method, the use, the lactic acid bacteria for use, or the composition according to any one of Items 1 to 7, wherein the mitochondrial-function-improving agent is a pharmaceutical or a quasi-drug.

Advantageous Effects of Invention

The present invention can provide a novel mitochondrial-function-improving agent. Specifically, mitochondrial function is improved by suppressing the production level of reactive oxygen species in mitochondria. In previous studies conducted by the present inventors, a method for preventing and treating viral diseases using an antiviral agent containing lactic acid bacteria has been proposed (PTL 1). However, to date, it has not been reported whether lactic acid bacteria capable of suppressing the production of reactive oxygen species are capable of improving mitochondrial function compared with lactic acid bacteria that do not have such an ability. Numerous factors are known to be involved in mitochondrial function, and these factors are intricately related. Therefore, the effect of the present invention, i.e., improving mitochondrial function, is unpredictable.

Brief Description of Drawings

Fig. 1 shows the results of Example 1 (2). (a): validation results of the effect of MitoSOX addition on cultured cells (cell detachment); (b): validation results of the effect of MitoROS 580 addition on cultured cells (cell detachment); and (c): results of detection and quantification of Antimycin A-induced ROS with MitoROS 580.

Fig. 2 shows the results of Example 1 (3). (a): validation results of ROS-inducing pro-inflammatory substances for use in a mitochondrial dysfunction model; and (b), (c), and (d): measurement results of ROS production levels 24 hours, 3 hours, and 30 minutes after the addition of candidate substances to cultured cells.

Fig. 3 shows the validation results of the effect of the addition of dead lactic acid bacteria on the ability to suppress the production of reactive oxygen species in mitochondria in Example 1 (5).

Fig. 4 shows the validation results of the effect of the addition of FFIG35 on the oxygen consumption rate in Example 2 (3).

Fig. 5 shows the validation results of the effect of the addition of FFIG35 on the ATP production level in Example 2 (4) .

Fig. 6 shows the validation results of the effect of the addition of FFIG35 on ROS production in mitochondria in porcine intestinal macrophages in Example 3.

Description of Embodiment

Mitochondrial-function-improving Agent

The present invention provides a mitochondrial-function-improving agent comprising lactic acid bacteria capable of suppressing the production of reactive oxygen species in mitochondria.

Ability to Suppress the Production of Reactive Oxygen Species in Mitochondria

Reactive oxygen species refer to oxygen species that have greater reactivity and are more active than naturally occurring ground state triplet oxygen. In the present invention, the term "reactive oxygen species" includes superoxide, hydrogen peroxide, hydroxyl radical, and singlet oxygen. In a typical embodiment of the present invention, lactic acid bacteria capable of suppressing the production of reactive oxygen species means lactic acid bacteria capable of suppressing the production of at least one of the reactive oxygen species mentioned above. Therefore, the production level of reactive oxygen species can be measured by detecting at least one selected from the group consisting of superoxide, hydrogen peroxide, hydroxyl radical, and singlet oxygen. Preferably, the production level of reactive oxygen species can be measured by detecting superoxide.

In viable cells, most reactive oxygen species are localized in mitochondria or present in the cytosol. In the present invention, the reactive oxygen species in mitochondria refer to reactive oxygen species localized in mitochondria.

The phrase "suppressing the production of reactive oxygen species" means having a tendency towards a reduction in the production level of reactive oxygen species. For example, when 1) cells obtained by adding an inflammation inducer to cultured cells to increase the production level of reactive oxygen species in mitochondria are compared with 2) cells obtained by adding lactic acid bacteria, which are an active ingredient in the present invention, to cultured cells and then adding an inflammation inducer to increase the production level of reactive oxygen species in mitochondria, and when the production level of reactive oxygen species is smaller in the latter 2), the lactic acid bacteria are considered to be capable of suppressing the production of reactive oxygen species.

In the present invention, target mitochondria for suppressing the production of reactive oxygen species are not particularly limited, and examples include mitochondria in cultured cells (including cells in tissue culture) and in biological tissues. In an embodiment of cultured cells, preferably, cells derived from the intestinal tract can be used, and more preferably, intestinal epithelial cells or intestinal macrophages can be used. In an embodiment of biological tissues, the target tissue is preferably the intestinal tract, and more preferably intestinal epithelium or intestinal macrophages. The organism from which the cells or tissues are derived is not particularly limited and is preferably a mammal. Examples of mammals include humans, pigs, monkeys, mice, rats, rabbits, cats, dogs, cows, horses, and sheep, with humans or pigs being preferred.

Lactic Acid Bacteria

In the present specification, lactic acid bacteria are bacteria that produce lactic acid through metabolism and that satisfy the following two conditions: being Gram-positive bacteria and being rod-shaped or spherical.

In a typical embodiment, the present invention uses lactic acid bacteria capable of suppressing the production of reactive oxygen species in mitochondria. The percentage of suppression of the production level of reactive oxygen species is not particularly limited. In one embodiment of the present invention, the production level of reactive oxygen species in mitochondria is preferably reduced to 70% to 90%, more preferably 80% to 90%, and even more preferably 80% to 85%. The value of the percentage of suppression of the production level of reactive oxygen species can be the arithmetic mean value of the percentages calculated from two or three or more measurement values, and it is preferable that a significant difference is found in Dunnett's test. That is, the p-value obtained in Dunnett's test is preferably $p < 0.05$, more preferably $p < 0.01$, and even more preferably $p < 0.001$.

The method for measuring the production level of reactive oxygen species in mitochondria is not particularly limited. Methods that use reagents and measuring devices known to be capable of measuring the production level of reactive oxygen species in mitochondria may be used; such known measurement methods may also be used after appropriate modification. From a throughput perspective, it is preferable to measure the production level of reactive oxygen species in mitochondria by using a fluorescence detection reagent. In a measurement method that uses a fluorescence detection reagent, for example, the production level of reactive oxygen species can be measured by detecting, with a fluorescence detection device, such as a plate reader or a flow cytometer, the emission intensity of fluorescence exhibited when the reagent added to the sample reacts with the reactive oxygen species in mitochondria. Examples of the fluorescence detection reagent include MitoROS 580 (AAT Bioquest) and MitoSOX (Invitrogen); MitoROS 580 can be preferably used. MitoROS 580 contains a superoxide-sensitive fluorescent dye that immediately migrates to mitochondria upon entering a viable cell, and thus can be used as a reagent for measuring the production level of reactive oxygen species in mitochondria.

More specifically, examples of lactic acid bacteria include bacteria of the genus *Ligilactobacillus,* the genus *Lactiplantibacillus,* the genus *Lactobacillus,* the genus *Amylolactobacillus,* the genus *Holzapfelia,* the genus *Bombilactobacillus,* the genus *Companilactobacillus,* the genus *Lapidilactobacillus,* the genus *Agrilactobacillus,* the genus *Schleiferilactobacillus,* the genus *Lacticaseibacillus,* the genus *Paralactobacillus,* the genus *Latilactobacillus,* the genus *Loigolactobacillus,* the genus *Dellaglioa,* the genus *Liquorilactobacillus,* the genus *Furfurilactobacillus,* the genus *Paucilactobacillus,* the genus *Limosilactobacillus,* the genus *Secundilactobacillus,* the genus *Levilactobacillus,* the genus *Fructilactobacillus,* the genus *Acetilactobacillus,* the genus *Apilactobacillus,* and the genus *Lentilactobacillus,* with bacteria of the genus *Ligilactobacillus* and the genus *Lactiplantibacillus* being preferred, and bacteria of the genus *Ligilactobacillus* being more preferred.

The bacteria of the genus *Ligilactobacillus* can preferably comprise *Ligilactobacillus salivarius.*

In one preferable embodiment, the lactic acid bacteria can comprise *Ligilactobacillus salivarius* with accession number NITE BP-03218 (FFIG35) or *Ligilactobacillus salivarius* with accession number NITE ABP-03958 (FFIG56), or both, and more preferably *Ligilactobacillus salivarius* with accession number NITE BP-03218 (FFIG35). These strains were respectively deposited on May 19, 2020, and August 4, 2023, with the Patent Microorganism Depository, National Institute of Technology and Evaluation (NITE) (Room No. 122, 2-5-8 Kazusakamatari, Kisarazushi, Chiba Prefecture, Japan, 292-0818) under the Budapest Treaty.

The lactic acid bacteria of the genus *Lactiplantibacillus* can preferably comprise *Lactiplantibacillus plantarum,* and more preferably the CS91 strain or the CS96 strain, or both. The CS91 and CS96 strains are stored in the laboratory of the inventors, and the CS91 and CS96 strains can be provided by the inventors to those skilled in the art as necessary.

The lactic acid bacteria in the present invention can be cultured according to known methods.

When culturing lactic acid bacteria using a medium, a known medium for culturing lactic acid bacteria can be used, or such a known medium can be used after appropriate modification. Examples of the medium include, but are not limited to, MRS medium (de Man, Rogosa, Sharpe, Becton Dickinson and Company). Alternatively, the medium may be a culture solution. An amino acid or a salt thereof, or a combination thereof, may be appropriately added to the medium. It is also possible to use food ingredients or food products instead of the medium.

To promote bacterial growth, the culture temperature is, for example, 15 to 60°C, preferably 25 to 50°C, and more preferably 35 to 40°C. The pH of the medium or culture solution is, for example, 4.8 to 6.8, and preferably 6.0 to 6.5. The culture time is, for example, 10 hours to 3 days, and preferably 16 hours to 24 hours. Lactic acid bacteria can be cultured under anaerobic conditions or under aerobic conditions. The culture here may be performed on a laboratory scale or on an industrial scale.

Improvement of Mitochondrial Function

Mitochondria perform cellular respiration using oxygen as the electron acceptor and produce adenosine triphosphate (ATP) in conjunction with the electron transport system. Therefore, mitochondrial function can be evaluated by measuring the activity of the electron transport system. Specifically, when mitochondrial function declines, the oxygen consumption rate (OCR) decreases. Conversely, when mitochondrial function is improved, the oxygen consumption rate recovers.

In the present invention, the method for measuring the oxygen consumption rate is not particularly limited. Examples include measurement methods that use an extracellular flux analyzer, a plate reader capable of detecting fluorescence, and the like, with a measurement method that use an extracellular flux analyzer being preferred. Examples of extracellular flux analyzers include an Agilent Seahorse XF24 analyzer (Seahorse Bioscience).

For the oxygen consumption rate, basal respiration capacity and maximal respiration capacity can be evaluated with the Agilent Seahorse XF24 analyzer. More specifically, the oxygen consumption rate can be measured and evaluated, for example, by the method described below in the Examples of the present application. In basal respiration, the mitochondrial oxygen consumption rate is preferably 105% or more, more preferably 110% or more, and even more preferably 120% or more, compared with a control to which the mitochondrial-function-improving agent of the present invention is not added. The upper limit of the oxygen consumption rate is not limited, and the upper limit of the mitochondrial oxygen consumption rate may be, for example, 140% or less, 130% or less, or 125% or less, compared with a control to which the mitochondrial-function-improving agent of the present invention is not added. In maximal respiration, the mitochondrial oxygen consumption rate is preferably 105% or more, and more preferably 110% or more, compared with a control to which the mitochondrial-function-improving agent of the present invention is not added. The upper limit of the oxygen consumption rate is not limited, and the upper limit of the mitochondrial oxygen consumption rate may be, for example, 130% or less, 120% or less, or 115% or less, compared with a control to which the mitochondrial-function-improving agent of the present invention is not added.

Eighty to ninety percent of ATP production in eukaryotes is from the mitochondrial electron transport system, and a decrease in intracellular ATP production reflects the activity of the electron transport system, which is an index of mitochondrial activity. Therefore, mitochondrial function can also be evaluated by directly measuring the intracellular ATP production level. That is, when mitochondrial function declines, the ATP production level decreases. Conversely, when mitochondrial function is improved, the ATP production level recovers.

In the present invention, the method for measuring the ATP production level is not particularly limited. Examples include a method of quantifying the ATP level in cultured cells based on a luciferase luminescence method. Examples of luminescent enzymes for use in the luciferase luminescence method include firefly luciferase, *Renilla* luciferase, and *Cypridina* luciferase, with firefly luciferase being preferred.

Therefore, the measurement is preferably performed using a plate reader capable of detecting chemiluminescence. Examples of the plate reader include ARVO X3 (PerkinElmer). The detection reagent is not particularly limited, and an ATP Assay Kit-Luminescence (DOJINDO) is preferably used. More specifically, the ATP production level can be measured and evaluated, for example, by the method described below in the Examples of the present application. The mitochondrial ATP production level is preferably 110% or more, and more preferably 115% or more, compared with a control to which the mitochondrial-function-improving agent of the present invention is not added. The upper limit of the ATP production level is not limited, and the upper limit of the mitochondrial ATP production level may be, for example, 140% or less, 130% or less, or 120% or less, compared with a control to which the mitochondrial-function-improving agent of the present invention is not added.

In the present invention, the active ingredient of the present invention, i.e., lactic acid bacteria themselves capable of suppressing the production of reactive oxygen species in mitochondria, may be used as a mitochondrial-function-improving agent, or the lactic acid bacteria may be used in the form of a composition in combination with various carriers (e.g., isotonic agents, chelating agents, stabilizers, pH adjusters, preservatives, antioxidants, solubilizing agents, and thickeners), food materials, and the like to an extent that does not impair the effects of the present invention.

Examples of isotonic agents include sugars, such as glucose, trehalose, lactose, fructose, mannitol, xylitol, and sorbitol; polyhydric alcohols, such as glycerin, polyethylene glycol, and propylene glycol; and inorganic salts, such as sodium chloride, potassium chloride, and calcium chloride. These isotonic agents can be used singly or in a combination of two or more.

Examples of chelating agents include edetates, such as disodium edetate, calcium disodium edetate, trisodium edetate, tetrasodium edetate, and calcium edetate, as well as ethylenediaminetetraacetate, nitrilotriacetic acid or its salt, sodium hexametaphosphate, and citric acid. These chelating agents can be used singly or in a combination of two or more.

Examples of stabilizers include sodium bisulfite.

Examples of pH adjusters include acids, such as hydrochloric acid, carbonic acid, acetic acid, and citric acid, alkali metal hydroxides, such as sodium hydroxide and potassium hydroxide, alkali metal carbonates or hydrogencarbonate, such as sodium carbonate, alkali metal acetates, such as sodium acetate, alkali metal citrates, such as sodium citrate, and bases, such as trometamol. These pH adjusters can be used singly or in a combination of two or more.

Examples of preservatives include sorbic acid, potassium sorbate, p-hydroxybenzoates, such as methyl p-hydroxybenzoate, ethyl p-hydroxybenzoate, propyl p-hydroxybenzoate, and butyl p-hydroxybenzoate, quaternary ammonium salts, such as chlorhexidine gluconate, benzalkonium chloride, benzethonium chloride, and cetylpyridinium chloride, alkyl polyaminoethyl glycine, chlorobutanol, polyquaternium, polyhexamethylene biguanide, and chlorhexidine. These preservatives can be used singly or in a combination of two or more.

Examples of antioxidants include sodium bisulfite, dried sodium sulfite, sodium pyrosulfite, and mixed tocopherol concentrate. These antioxidants can be used singly or in a combination of two or more.

Examples of solubilizing agents include sodium benzoate, glycerin, D-sorbitol, glucose, propylene glycol, hydroxypropyl methylcellulose, polyvinylpyrrolidone, macrogol, and D-mannitol. These solubilizing agents can be used singly or in a combination of two or more.

Examples of thickeners include polyethylene glycol, methyl cellulose, ethyl cellulose, carmellose sodium, xanthan gum, sodium chondroitin sulfate, hydroxyethyl cellulose, hydroxypropyl cellulose, hydroxypropyl methylcellulose, polyvinylpyrrolidone, and polyvinyl alcohol. These thickeners can be used singly or in a combination of two or more.

The mitochondrial-function-improving agent of the present invention may further comprise components known to improve mitochondrial function. Examples of such components include taurine, vitamin C, and catechins. These components can be used singly or in a combination of two or more.

In the present invention, target mitochondria of the mitochondrial-function-improving agent are not particularly limited, and examples include mitochondria in cultured cells (including cells in tissue culture) and in biological tissues. In an embodiment of cultured cells, preferably, cells derived from the intestinal tract can be used, and more preferably, intestinal epithelial cells or intestinal macrophages can be used. In an embodiment of biological tissues, the target tissue is preferably the intestinal tract, and more preferably intestinal epithelium or intestinal macrophages. The organism from which the cells or tissues are derived is not particularly limited and is preferably a mammal. Examples of mammals include humans, pigs, monkeys, mice, rats, rabbits, cats, dogs, cows, horses, and sheep, with humans or pigs being preferred.

The mitochondrial-function-improving agent of the present invention can also be used as an experimental reagent in cell experiments (including tissue culture experiments) or in vivo cell-based experiments. Further, the method of providing the mitochondrial-function-improving agent of the present invention is not particularly limited; the mitochondrial-function-improving agent of the present invention can be used, for example, as a food, a beverage, a pharmaceutical, or a quasi-drug.

In an embodiment of a food and a beverage, the mitochondrial-function-improving agent of the present invention can be prepared in a desired form either directly or in combination with other food materials or additive ingredients. Examples of such foods and beverages include general foods and beverages, as well as foods for specified health uses, dietary supplements, functional foods, and foods for the sick.

Examples of the embodiment of such foods and beverages include, but are not particularly limited to, supplements, such as capsules (soft capsules and hard capsules), tablets, granules, powders, jellies, and liposome formulations; beverages, such as nutritional drinks, fruit juice beverages, carbonated beverages, and lactic acid beverages; and confectioneries, such as dumplings, ice cream, sherbets, gummies, and candies. Among these foods and beverages, beverages and supplements are preferred.

The content of the lactic acid bacteria capable of suppressing the production of reactive oxygen species in mitochondria as the active ingredient of the present invention varies depending on the administration route, patient's age, body weight, symptoms, and the like, and cannot be uniformly defined. The amount can be set so that the daily intake of compound (I) for an adult is usually about 10 to 5000 mg, and more preferably about 100 to 1000 mg. In an embodiment of the composition, the content of the lactic acid bacteria capable of suppressing the production of reactive oxygen species in mitochondria in the composition is not particularly limited and can be appropriately set, for example, from conditions, such as 90 mass% or more, 70 mass% or more, 50 mass% or more, 30 mass% or more, 10 mass% or more, 5 mass% or more, and 1 mass% or more.

When the mitochondrial-function-improving agent of the present invention is provided as a pharmaceutical or a quasi-drug, the dosage form is not particularly limited; examples include oral administration agents, such as tablets, pills, capsules, powders, granules, syrups, and sublingual tablets; parenteral administration agents, such as injections (e.g., intravenous injection, intramuscular injection, and local injection), gargles, infusions, external preparations (ointments, creams, patches, and inhalants), and suppositories; and various other dosage forms. Among the dosage forms mentioned above, preferred are, for example, injections, oral administration agents, and external preparations, with inhalants (intratracheal preparations) being particularly preferred.

In an embodiment of the pharmaceutical or quasi-drug, the content of the lactic acid bacteria capable of suppressing the production of reactive oxygen species in mitochondria as the active ingredient of the present invention varies depending on the administration route, patient's age, body weight, symptoms, and the like, and cannot be uniformly defined. The content can be set such that the daily dose of compound (I) for an adult is usually about 10 to 5000 mg, and more preferably about 100 to 1000 mg.

The present invention will be described in more detail below with reference to Examples; however, the present invention is not limited to these Examples.

Examples

In the Examples, unless otherwise specified, special grade reagents or first grade reagents produced by Wako Pure Chemical Industries, Ltd. (Osaka, Japan) were used. All water used was MilliQ water (Merck Millipore, Germany).

Example 1

Validation of the Ability of Lactic Acid Bacteria to Suppress the Production of Reactive Oxygen Species in Mitochondria

(1) Porcine Intestinal Epithelial Cells (INV1-3)

In this Example, the ability of lactic acid bacteria to suppress the production of reactive oxygen species in mitochondria was validated using porcine intestinal epithelial cells (INV1-3). The INV1-3 cells are cloned from the small intestine of Duroc piglets and reflect a polymorphism (gain-of-function) in the NOD2 gene in the genome.

For culture of the INV1-3 cells, collagen (type I)-coated 250 mL flasks (Sumiron) and DMEM/F-12 medium (10% FCS, 1% penicillin-streptomycin, 1% ITS (100×), 0.05% rec Human EGF (10 μg/mL), high glucose, L-glutamine, 0.11 ng/mL sodium pyruvate; Thermo Fisher Scientific, USA) (referred to below as "DMEM/F-12 medium (+FCS)") were used.

The cells were maintained in an incubator at 37°C with 5% $CO_2$. Upon reaching 80-90% confluence, the cells were washed once with PBS, treated with epithelial buffer (0.1 M $Na_2HPO_4$/12$H_2$O, 0.45 M sucrose, 0.36% EDTA/4Na, BSA) (37°C, 5 minutes), and then detached from the flasks with trypsin buffer (0.25% trypsin, 0.02% EDTA in PBS) (37°C, 5 minutes). The cells were collected by centrifugation (12000 rpm, 5 minutes, 4°C), counted, and then seeded into 250 mL flasks at $3.5 \times 10^5$ cells per flask. The day of seeding was defined as day 0 of culture, and on day 2 of culture, the culture supernatant was aspirated and removed with an aspirator, and 10 mL of fresh DMEM/F-12 medium was added for culture. In this Example, the cells between passage numbers 8 and 18 were used.

(2) Reagent for Measuring the Production Level of Reactive Oxygen Species in Mitochondria

Two candidates, MitoSOX and MitoROS 580, were analyzed to determine whether they could be used in plate assays that allow simultaneous analysis of multiple samples, and whether they could confirm the effects of long-term stimulation by food ingredients.

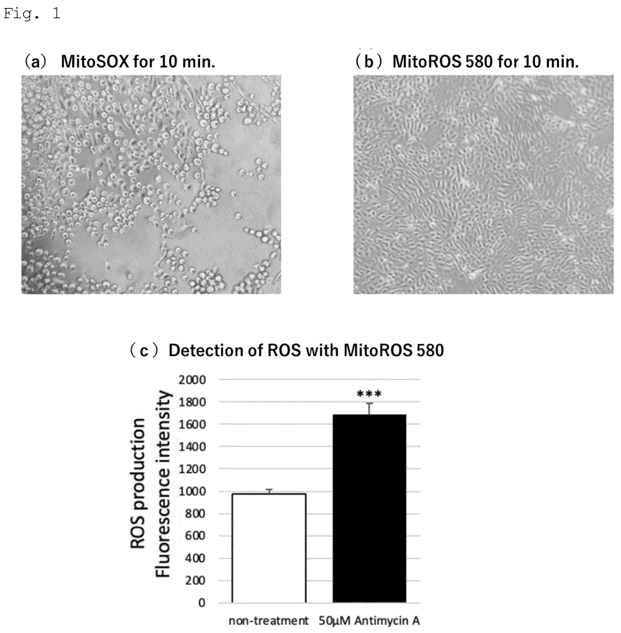

In order to confirm the effects of the reagents for measuring the ROS production level (MitoSOX and MitoROS 580) on the INV1-3 cells, each reagent was individually added to the cells. After incubation at 37°C for 10 minutes, the cells were observed under a microscope. Before addition, MitoSOX and MitoROS 580 were each dissolved in 50 µL of DMSO and diluted 1/500 with assay buffer. As a result, MitoSOX caused cell detachment as shown in Fig. 1(a), whereas MitoROS 580 did not cause cell detachment as shown in Fig. 1(b).

Next, in order to validate the ability of MitoROS 580 to detect ROS in mitochondria in INV1-3, Antimycin A, which is known as a ROS production inducer, was added to INV1-3 to promote ROS production. Antimycin A was added to a concentration of 50 µM in accordance with the protocol of MitoROS 580. Thereafter, MitoROS 580 was added to INV1-3. After incubation at 37°C for 1 hour, the fluorescence intensity (excitation: 540 nm, emission: 590 nm, cutoff: 570 nm) was measured with ARVO X3 (PerkinElmer) in bottom-read mode. Before addition to the cells, MitoROS 580 was dissolved in 50 µL of DMSO and diluted 1/500 with assay buffer. A non-treatment sample was prepared in the same manner except that Antimycin A was not added. The fluorescence intensity of the sample to which Antimycin A was added and the fluorescence intensity of the non-treatment sample were measured. The same experiment from the cell culture to the fluorescence intensity measurement as described above was independently performed 3 times (3 wells).

The ratio between the average value of the measured fluorescence intensity of the non-treatment sample (n = 3) and the average value of the measured fluorescence intensity of the sample to which Antimycin A was added (n = 3) was calculated. As shown in Fig. 1(c), the results confirmed that MitoROS 580 was capable of detecting Antimycin A-induced ROS.

Based on the above, the MitoROS 580 fluorescent reagent was selected as the reagent for measuring the production level of reactive oxygen species in mitochondria. Additionally, it was determined that MitoROS 580 was dissolved in 50 µL of DMSO and diluted 1/500 with assay buffer before adding to the cells.

(3) Analysis of ROS-inducing pro-inflammatory Substance for Use in Mitochondrial Dysfunction Model

To determine a mitochondrial ROS-inducing pro-inflammatory substance for use in a model representing a state in which mitochondrial function is reduced in intestinal epithelial cells (INV1-3), cell viability and ROS production-inducing capability were tested after the addition of candidate substances, and the concentration and the duration of action were analyzed.

Three types of candidate substances were used: dextran sulfate sodium salt (DSS), which is used to create inflammatory bowel disease models; dead *Fusobacterium nucleatum*, whose proliferation is confirmed during the development of inflammatory bowel disease; and lipopolysaccharide (LPS), which is used in immune-related inflammation induction models. The effective concentration of each candidate substance was analyzed by evaluating cell viability using a Cell Counting Kit-8 (Dojindo, Kumamoto) after 24 hours of stimulation.

Specifically, the INV1-3 cells were seeded in a collagen Type I-coated 96-well plate (SUMILON) at $3.0 \times 10^4$ cells/mL and cultured for 4 days. After replacing the medium, DSS, LPS, and dead *Fusobacterium nucleatum* were individually added and cultured at 37°C for 24 hours. Thereafter, the cells were washed thoroughly with PBS, and 105 µL of DMEM/F-12 medium (+FCS) containing 8% Cell Counting Kit-8 (Dojindo) was added, followed by incubation at 37°C for 2.5 hours. The absorbance (450 nm) was then measured using ARVO X3 (PerkinElmer). A non-treatment sample was prepared in the same manner except that the ROS-inducing pro-inflammatory substances were not added.

The same experiment from the cell culture to the absorbance measurement as described above was independently performed 3 times (3 wells).

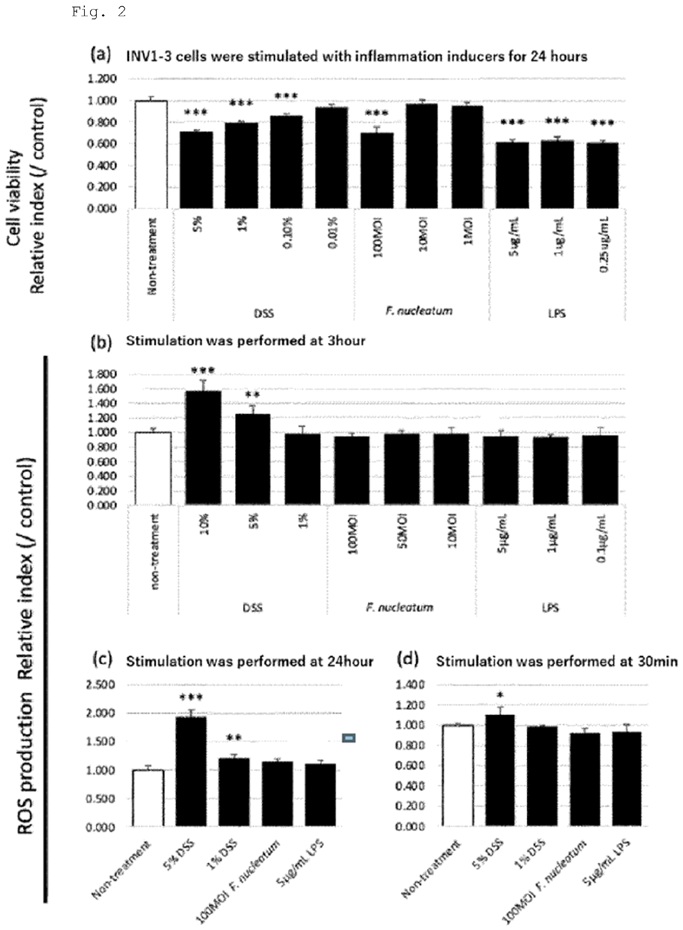

The ratios between the average values of the absorbance of the samples to which each ROS-inducing pro-inflammatory substance was added (n = 3) and the average value of the absorbance of the non-treatment sample (n = 3) were calculated and defined as the cell viability. The change in cell viability upon 24-hour exposure is shown. The cell viability was reduced to 80 to 90% (relative to Control) with DSS at 1% or more, dead *F. nucleatum* at an MOI of 100, or LPS at a concentration of 0.1 µg/mL or more (data for LPS concentration of 0.1 µg/mL not shown) (Fig. 2(a)).

Using these concentrations as a reference, the ROS production levels were measured at 24 hours, 3 hours, and 30 minutes after the addition of the mitochondrial ROS-inducing pro-inflammatory candidate substances, as shown in Figs. 2(b) to (d). The ROS production levels were measured using ROS 580 and ARVO X3 in the same manner as in Example 1 (2).

The same experiment from the cell culture to the fluorescence intensity measurement as described above was independently performed 3 times (3 wells).

The results revealed that among the candidate substances, only DSS increased the mitochondria-derived ROS production level. Specifically, as shown in Figs. 2(b) to (d), an increase in the ROS production level was confirmed when 24-hour stimulation was performed with 1-5% DSS and when 30-minute or 3-hour stimulation was performed with 5% DSS.

Based on the above, DSS was selected as the stimulant for use in the model of mitochondrial dysfunction in the INV1-3 cells; the concentration was set to 5% and the exposure time was set to 3 hours.

(4) Preparation of Strains for Use and Preparation of Dead Bacterial Cells

For use as candidate substances capable of suppressing the production of reactive oxygen species in mitochondria, the following lactic acid bacteria were cultured: *Ligilactobacillus salivarius* (FFIG35) (Accession No. NITE BP-03218), *Ligilactobacillus salivarius* (FFIG56) (Accession No. NITE ABP-03958), *Ligilactobacillus salivarius* (FFIG110), *Ligilactobacillus salivarius* (FFIG130), *Lactiplantibacillus plantarum* (CS91), and *Lactiplantibacillus plantarum* (CS96).

The strains used were isolated from pigs in the inventors' laboratory. Table 1 shows the origin and genus name of the strains used. 30% glycerol stock samples from the inventors' laboratory were applied to MRS agar medium with an inoculation loop.

Table 1

| Candidate bacteria | Solvent | Mfr. or Origin |
|---|---|---|
| Ligilactobacillus salivarius (FFIG35) | PBS | Ieum pyer's patch |
| Ligilactobacillus salivarius (FFIG56) | PBS | jejunum |
| Ligilactobacillus salivarius (FFIG110) | PBS | ileum |
| Ligilactobacillus salivarius (FFIG130) | PBS | Porcine ileal |
| Lactiplantibacillus plantarum (CS91) | PBS | Colostrum |
| Lactiplantibacillus plantarum (CS96) | PBS | Colostrum |

A single colony of the lactic acid bacteria was picked with a sterile toothpick, inoculated into 1 mL of MRS liquid medium, and cultured at 37°C for 24 hours. Subsequently, the culture solution was stirred, and 20 $\mu$L of the resulting product was added to 1 mL of MRS liquid medium and cultured at 37°C for one day. Similarly, the culture solution was stirred, and 200 $\mu$L of the resulting product was added to 10 mL of MRS liquid medium and cultured at 37°C for 16 hours.

Dead lactic acid bacteria were prepared according to the following procedure.

The culture solution was centrifuged (6000 rpm, 5 minutes). The supernatant was discarded, 5 mL of PBS was added, and the mixture was stirred until the pellet disappeared, followed by centrifugation (6000 rpm, 5 minutes). The supernatant was discarded, 5 mL of PBS was added, and the mixture was stirred. The resulting product was heat-treated for 90 minutes in a water bath preheated to 70°C. Subsequently, centrifugation was performed (6000 rpm, 5 minutes), the supernatant was discarded, 6 mL of PBS was added, and the mixture was stirred. Then, 1 mL of the resulting product was aliquoted and diluted 2-, 10-, and 50-fold with PBS. Ten microliters of the sample was placed on a bacterial counting chamber (AS ONE), covered with a cover glass, pressed until the cells stopped moving, and examined under a microscope. The bacterial count was determined, and the total bacterial count was calculated based on the following equation.

Total bacterial count C/mL = Measured count c $\times$ 250000 $\times$ [Dilution ratio]

After measuring the bacterial count, the resulting product was adjusted to a concentration of $2.5 \times 10^9$ cells/mL with PBS, aliquoted into micro-tubes, and the bacterial solution was stored frozen at -20°C.

(5) Validation of Ability to Suppress the Production of Reactive Oxygen Species in Mitochondria

INV1-3 (100 $\mu$L) was seeded in an XF24 cell culture plate (Agilent Technologies) at a concentration of $3.0 \times 10^4$ cells/mL and cultured at 37°C for 24 hours to allow for adhesion. Twenty-four hours after the start of culture, 150 $\mu$L of DMEM/F-12 medium (+FCS) was added to adjust the volume to 250 $\mu$L. The medium was replaced 72 hours after the start of culture; then, 96 hours after the start of culture, the candidate substances, the dead lactic acid bacteria, prepared in Example 1 (4) were added to the INV1-3 cells and cultured at 37°C for 24 hours. The dead bacterial cells were added at a multiplicity of infection (MOI) of 100 because a density of 100 MOI would clearly not significantly affect cell viability according to the previous study of the inventors' laboratory.

Subsequently, the INV1-3 cells were washed with PBS, DSS was added to a concentration of 5%, and the cells were exposed for 3 hours. A control sample was prepared in the same manner except that the candidate substances, the dead lactic acid bacteria, prepared in Example 1 (4) were not added.

Subsequently, the fluorescence intensity of the samples to which the dead lactic acid bacteria were added and the fluorescence intensity of the control sample were measured using ROS 580 and ARVO X3 in the same manner as in Example 1 (2).

The same experiment from the cell culture to the fluorescence intensity measurement as described above was independently performed 6 times (6 wells).

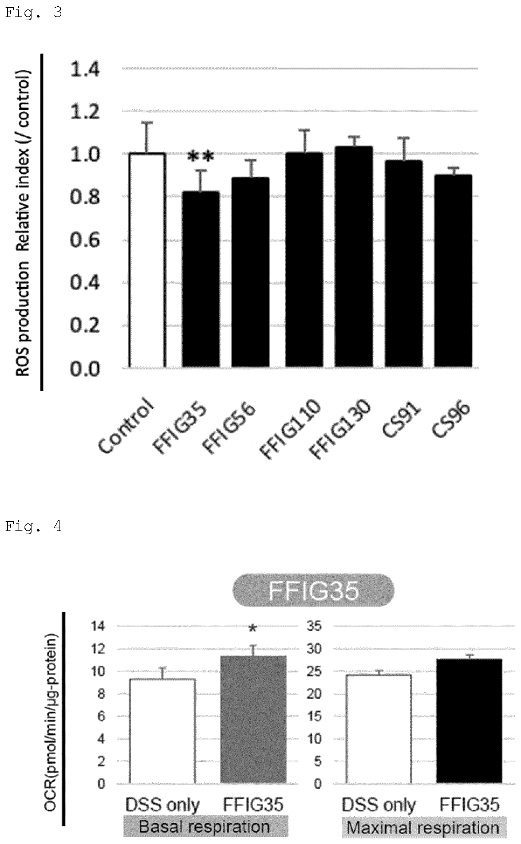

The ratio between the average value of the measured fluorescence intensity of the control sample (n = 6) and the average value of the measured fluorescence intensity of each of the samples (n = 6) was calculated and defined as the ROS production level in mitochondria of each sample (Table 2 and Fig. 3) .

Table 2

| Sample | Average | ROS production level | Standard deviation (SD) |
|---|---|---|---|
| Control | 681.927 | 1.000 | 0.148 |
| FFIG35 | 559.830 | 0.821 | 0.102 |
| FFIG56 | 605.083 | 0.887 | 0.083 |
| FFIG110 | 681.617 | 1.000 | 0.114 |
| FFIG130 | 704.417 | 1.033 | 0.046 |
| CS91 | 659.826 | 0.968 | 0.106 |
| CS96 | 612.002 | 0.897 | 0.036 |

Example 2

Validation of Mitochondrial-function-improving Effect of Lactic Acid Bacteria Capable of Suppressing the Production of Reactive Oxygen Species in Mitochondria

(1) Cells Used and Culture Conditions

The INV1-3 cells, which are immortalized porcine intestinal epithelial cells, were used as in Example 1. The culture method is also in accordance with Example 1 (1).

(2) Test Strains and Preparation Method

*Ligilactobacillus salivarius* (FFIG35), which showed a remarkable reduction in the ROS production level in Example 1, was used. A dead cell sample was prepared in the same manner as in Examples 1 (4) and (5).

(3) Measurement of Mitochondrial Oxygen Consumption Rate using Extracellular Flux Analyzer

- Preparation of Measurement Sample

INV1-3 (100 µL) was seeded in an XF24 cell culture plate (Agilent Technologies) at a concentration of $3.0 \times 10^4$ cells/mL and cultured at 37°C for 24 hours to allow for adhesion.

Twenty-four hours after the start of culture, 150 µL of DMEM/F-12 medium (+FCS) was added to adjust the volume to 250 µL. Seventy-two hours after the start of culture, the medium was replaced, and 96 hours after the start of culture, dead FFIG35 cells were added and cultured at 37°C for 24 hours. The dead FFIG35 cells were added at an MOI of 100. Thereafter, the INV1-3 cells were washed with PBS, DSS was added to a concentration of 5%, and the cells were exposed for 3 hours.

A DSS-only sample was prepared in the same manner except that the dead FFIG35 cells were not added.

Measurement of Oxygen Consumption Rate (OCR)

The 5% DSS-containing medium in each well was replaced with 500 µL of XF assay medium (containing 25 mM glucose, 2 mM glutamine, and 1 mM sodium pyruvate, serum-free, Agilent Technologies). Then, 10 µM Oligomycin, 20 µM FCCP, and 5 µM Rot/AA, which are reagents of a Cell Mito Stress Test Kit (Agilent Technologies), were added to ports A, B, and C, respectively, and mitochondrial oxygen consumption rate (basal respiration) and maximal respiratory capacity (maximal respiration) in a steady state were measured using an Agilent Seahorse XF24 Analyzer (Seahorse Bioscience).

Next, the protein content was measured using a BCA Protein Assay Kit (Thermo Fisher Scientific).

For the DSS-only sample, the same experiment was performed 5 times (5 wells), and for the sample to which dead FFIG35 cells were added, the same experiment was performed 4 times (4 wells).

Taking the protein content of a single well of the DSS-only sample as 1, the measured oxygen consumption rates in basal respiration and the measured oxygen consumption rates in maximal respiration were corrected based on the protein content. The average values of the measured oxygen consumption rates in basal respiration and maximal respiration after correction (DSS-only: n = 5, FFIG35: n = 4) were defined as the oxygen consumption rates in basal respiration and maximal respiration, respectively.

The data were analyzed for oxygen consumption rates (OCR) using Seahorse Wave Desktop software (Agilent Technologies) (Table 3 and Fig. 4).

Table 3

| | Sample | Oxygen consumption rate | Standard deviation (SD) |
|---|---|---|---|
| Basal Respiration | DSS-only | 9.304 | 1.231 |
| | FFIG35 | 11.313 | 1.053 |
| Maximal Respiration | DSS-only | 24.201 | 2.923 |
| | FFIG35 | 27.692 | 3.632 |

(4) Measurement of Mitochondrial ATP Production Level using Plate Reader

- Preparation of Measurement Sample

A 96-well plate (SUMILON) was seeded with 100 $\mu$L per well of a cell suspension at $3.0 \times 10^4$ cells/mL, and the cells were cultured at 37°C for 24 hours to allow for adhesion.

DMEM/F-12 medium (+FCS) was added 24 hours after the start of culture. The medium was replaced 72 hours after the start of culture, and the dead FFIG35 cells were added 96 hours after the start of culture, followed by culturing at 37°C for 24 hours. The dead FFIG35 cells were added at an MOI of 100. Thereafter, the INV1-3 cells were washed with PBS, DSS was added to a concentration of 5%, and the cells were exposed for 3 hours. A DSS-only sample was prepared in the same manner except that the dead FFIG35 cells were not added. A non-treatment sample was prepared in the same manner except that DSS was not added and the dead FFIG35 cells were not added.

- Measurement of ATP Production Level

The working solution of the ATP Assay Kit-Luminescence (DOJINDO) was prepared in accordance with the recommended protocol.

The 5% DSS-containing medium was removed, washing was performed twice with PBS, and then 50 $\mu$L of DMEM/F-12 medium (-FCS) was added to each well. ATP standard solutions (5, 2.5, 0.625, 0.313, 0.078, and 0 $\mu$mol/L) were added to cell-free wells at 50 $\mu$l per well. Then, the working solution was added at 50 $\mu$L per well, and shaking was performed at 25°C for 2 minutes in an ARVO X3 plate reader, followed by incubation at 25°C for 10 minutes as is without opening. The luminescence (RLU) was recorded once for 0.1 second.

Subsequently, the protein content of each well was measured using a BCA Protein Assay Kit (Thermo Fisher Scientific).

The same experiment from the cell culture to the protein content measurement as described above was performed 6 times (6 wells).

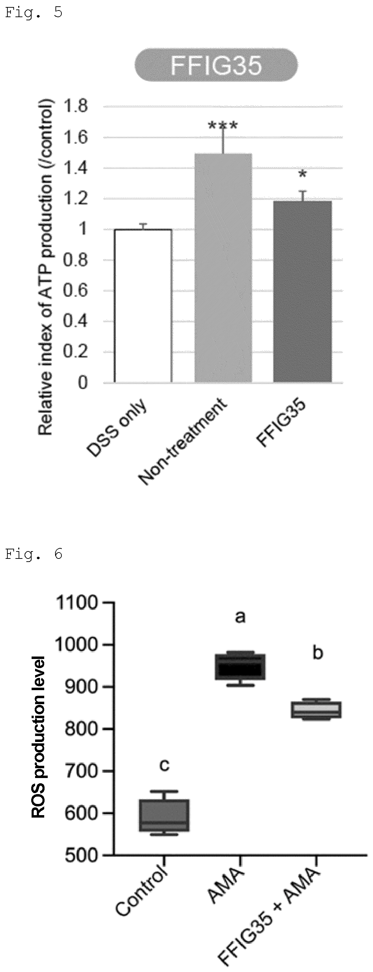

The measured emission intensity of each sample was corrected based on the protein content of each sample, with the protein content of the DSS-only sample taken as 1. Furthermore, the ratio between the average value of the measured emission intensity after correction of the non-treatment sample or the sample to which FFIG35 was added (n = 6) and the average value of the measured emission intensity of the DSS-only sample (n = 6) was calculated and defined as the cellular ATP production level (Table 4 and Fig. 5).

Table 4

| Sample | Average | ATP production level | Standard deviation (SD) |
|---|---|---|---|
| DSS-only | 35035.309 | 1.000 | 0.037 |
| Non-treatment | 52391.285 | 1.495 | 0.168 |

(continued)

| Sample | Average | ATP production level | Standard deviation (SD) |
|---|---|---|---|
| FFIG35 | 41427.772 | 1.182 | 0.067 |

Example 3

ROS Production-suppressing Effect of FFIG-35 in Intestinal Macrophages

Porcine intestinal macrophages were stimulated with post-immunobiotics (FFIG35) (48 hr). Specifically, porcine intestinal macrophages (Takenouchi et al., Front Vet Sci. 9:919077. 2022) were used. The porcine intestinal macrophages (100 $\mu$L) were seeded in an XF24 cell culture plate (Agilent Technologies) at a concentration of $6.0 \times 10^4$ cells/mL and cultured at 37°C for 72 hours. The medium was replaced, and the dead FFIG35 cells prepared according to the method described in Example 1 (4) were added to the porcine intestinal macrophages and cultured at 37°C for 24 hours. The dead FFIG35 cells were added at an MOI of 100.

Subsequently, stimulation was performed with Antimycin A (AMA), which is an inhibitor of mitochondrial electron transport chain complex III. Specifically, the porcine intestinal macrophages were washed with PBS, AMA was added to a concentration of 50 $\mu$M, and the macrophages were exposed for 3 hours. A control sample was prepared in the same manner except that the dead FFIG35 cells were not added.

The ROS production level in mitochondria was measured using mitROS. Fig. 6 shows the results.

The results demonstrate that AMA stimulation induced ROS production. On the other hand, when pre-stimulation was performed with FFIS35, AMA-induced ROS production was significantly suppressed (significant differences were observed between opposite signs). This fact suggests that FFIG35 is capable of suppressing ROS production not only in intestinal epithelial cells, but also in intestinal macrophages.

**Claims**

1. A mitochondrial-function-improving agent comprising lactic acid bacteria capable of suppressing the production of reactive oxygen species in mitochondria.

2. The mitochondrial-function-improving agent according to claim 1, wherein the lactic acid bacteria reduce the production level of reactive oxygen species in mitochondria to 70% to 90%.

3. The mitochondrial-function-improving agent according to claim 1, wherein the lactic acid bacteria comprise *Ligilactobacillus salivarius.*

4. The mitochondrial-function-improving agent according to claim 1, wherein the lactic acid bacteria comprise *Ligilactobacillus salivarius* with accession number NITE BP-03218 or *Ligilactobacillus salivarius* with accession number NITE ABP-03958, or both.

5. The mitochondrial-function-improving agent according to claim 1, wherein the lactic acid bacteria are capable of suppressing the production of reactive oxygen species in mitochondria in intestinal epithelial cells.

6. The mitochondrial-function-improving agent according to claim 1, wherein the improvement of mitochondrial function includes at least one selected from the group consisting of an increase in ATP production level and an increase in mitochondrial oxygen consumption rate.

7. The mitochondrial-function-improving agent according to any one of claims 1 to 6, which is a food or a beverage.

8. The mitochondrial-function-improving agent according to any one of claims 1 to 6, which is a pharmaceutical or a quasi-drug.

Fig. 1

(a) MitoSOX for 10 min.

(b) MitoROS 580 for 10 min.

(c) Detection of ROS with MitoROS 580

Fig. 2

(a) INV1-3 cells were stimulated with inflammation inducers for 24 hours

(b) Stimulation was performed at 3hour

(c) Stimulation was performed at 24hour

(d) Stimulation was performed at 30min

Fig. 3

Fig. 4

Fig. 5

Fig. 6

## INTERNATIONAL SEARCH REPORT

| International application No. |
| --- |
| **PCT/JP2024/031324** |

| A. | CLASSIFICATION OF SUBJECT MATTER |
| --- | --- |

*A23L 33/135*(2016.01)i; *A61K 35/744*(2015.01)i; *A61P 3/10*(2006.01)i; *A61P 25/00*(2006.01)i; *A61P 35/00*(2006.01)i;
*A61P 43/00*(2006.01)i; *C12N 1/20*(2006.01)i
FI:  A23L33/135; A61K35/744; A61P43/00 111; A61P25/00; A61P3/10; A61P35/00; C12N1/20 E

According to International Patent Classification (IPC) or to both national classification and IPC

| B. | FIELDS SEARCHED |
| --- | --- |

Minimum documentation searched (classification system followed by classification symbols)

A23L33/135; A61K35/744; A61P3/10; A61P25/00; A61P35/00; A61P43/00; C12N1/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2022-112387 A (KIRIN HOLDINGS CO., LTD.) 02 August 2022 (2022-08-02) claim 1, paragraph [0069] | 1-2, 6-8 |
| Y | | 3-5 |
| X | KUMAR, Arun et al. A Potential Probiotic Lactobacillus plantarum JBC5 Improves Longevity and Healthy Aging by Modulating Antioxidative, Innate Immunity and Serotonin-Signaling Pathways in Caenorhabditis elegans. Antioxidants. 28 January 2022, vol. 11, article no. 268, DOI: 10.3390/antiox11020268 abstract | 1-2, 6-8 |
| Y | | 3-5 |

✓ Further documents are listed in the continuation of Box C.     ✓ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **12 November 2024** | **19 November 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
| --- |
| **PCT/JP2024/031324** |

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| X | SHIN, Min Jae et al. Screening for Lactic Acid Bacterial Strains as Probiotics Exhibiting Anti-inflammatory and Antioxidative Characteristic Via Immune Modulation in HaCaT Cell. Probiotics and Antimicrobial Proteins. 20 February 2023, vol. 15, no. 6, pp. 1665-1680. DOI: 10.1007/s12602-023-10048-8 <br> p. 1677, left column | 1-2, 6-8 |
| Y | | 3-5 |
| X | 小林 麻己人, "乳酸菌がもつ抗酸化作用の分子基盤解明", コスメトロジー研究報告 [online], 2022, vol. 30, pp. 127-130, [retrieved on 12 November 2024], Internet:URL<https://www.kose-cosmetology.or.jp/research_report/archives/2022/fullVersion/Cosmetology_Vol30_2022_p127-130_Kobayashi_M.pdf>, (KOBAYASHI, Makoto, Elucidation of the molecular basis of antioxidant effects of lactic acid bacteria, Cosmetology) <br> 3.3. Changes in gene expression by treatment with H61 strain | 1-2, 6-8 |
| Y | | 3-5 |
| X | 小畠 英史ら, Lactobacillus gasseri SBT2055による抗酸化ストレス作用の機構解明, BMB2015要旨集, 2015, 2P0225, (KOBATAKE, Eiji et al.), non-official translation (Elucidation of the mechanism of antioxidant stress action by Lactobacillus gasseri SBT2055, Abstracts of BMB2015) <br> abstract | 1-2, 6-8 |
| Y | | 3-5 |
| Y | WO 2022/164784 A1 (SEED HEALTH INC.) 04 August 2022 (2022-08-04) <br> claim 16, p. 23 | 3-5 |

Form PCT/ISA/210 (second sheet) (July 2022)

## INTERNATIONAL SEARCH REPORT
### Information on patent family members

International application No.

**PCT/JP2024/031324**

| Patent document cited in search report | | | Publication date (day/month/year) | Patent family member(s) | | | Publication date (day/month/year) |
|---|---|---|---|---|---|---|---|
| JP | 2022-112387 | A | 02 August 2022 | (Family: none) | | | |
| WO | 2022/164784 | A1 | 04 August 2022 | JP | 2024-505003 | A | |
| | | | | US | 2024/0082165 | A1 | |
| | | | | EP | 4284383 | A1 | |
| | | | | CN | 117042779 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021241728 A

- JP 2920818 B

**Non-patent literature cited in the description**

- *Biomedicines*, 2021, vol. 9, 216
- *Cell*, 2016, vol. 166, 555-566
- *The World Journal of Men's Health*, 2022, vol. 40, 399

- *International Journal of Molecular Sciences*, 2021, vol. 22, 6479